(19) Europäisches Patentamt
European Patent Office
Office européen des brevets

(11) Publication number: 0 310 312 A2

(12) # EUROPEAN PATENT APPLICATION

(21) Application number: 88308877.5

(22) Date of filing: 23.09.88

(51) Int. Cl.4: C07F 9/24 , C07H 21/04 , C12Q 1/68

(30) Priority: 02.10.87 US 104330

(43) Date of publication of application: 05.04.89 Bulletin 89/14

(84) Designated Contracting States: AT BE CH DE ES FR GB GR IT LI LU NL SE

(71) Applicant: M.L. TECHNOLOGY VENTURES, L.P.
c/o Merrill Lynch R & D Management Inc.
North Tower World Financial Center
New York New York 10281-1201(US)

(72) Inventor: Arnold, Lyle John
5439 Noah Way
San Diego California 92117(US)
Inventor: Bhatt, Ram Saroop
3575 San Toro Way
San Diego California 92130(US)

(74) Representative: Goldin, Douglas Michael et al
J.A. KEMP & CO. 14, South Square Gray's Inn
London WC1R 5EU(GB)

(54) **Non-nucleotide reagents for substituting termini of oligonucleotides.**

(57) A reagent which can couple with the 5′ hydroxy position of a nucleotide, typically the terminal nucleotide in a nucleotide multimer, under the same conditions used in known standard solid-phase nucleotide multimer synthesis. When so coupled, the reagent produces a substituted nucleotide multimer with a protected linking moiety which is deprotected under the same alkaline conditions typically used to deprotect nucleotide exocyclic amines of a completed synthetic nucleotide multimer. A single one of the class of reagents can be so coupled to any nucleotide, and since the reagents have a non-nucleotide skeleton, the resulting substituted nucleotide multimer does not have an additional nucleotide which could potentially interfere with hybridization with a target nucleotide sequence.

EP 0 310 312 A2

# NON-NUCLEOTIDE REAGENTS FOR SUBSTITUTING TERMINI OF OLIGONUCLEOTIDES

## FIELD OF THE INVENTION

This invention relates to reagents of a type which are suitable for preparing a 5′ hydroxy substituted nucleotide, the substituent of which can then be conveniently deprotected under the same alkaline conditions used to deprotect nucleotide exocyclic amines during standard solid-phase nucleotide multimer synthesis, to leave a linking group to which can then be attached useful moieties, for example a label or an intercalator.

## TECHNOLOGY REVIEW

In clinical research and diagnosis, a known technique for determining the presence of a particular nucleotide sequence (the "target nucleotide sequence" or simply the "target sequence") in either RNA or DNA polynucleotides, is to perform a nucleic acid hybridization assay. In such an assay, a nucleotide multimer probe (typically an oligonucleotide probe) is chosen which has a nucleotide sequence complimentary to at least a portion of the target sequence. Typically, the probe is labelled, that is, it is provided with an atom or a group linked thereto, the presence of which can be readily detected. When the labelled probe is exposed to a test sample suspected of containing the target nucleotide sequence, under hybridizing conditions, the two will hybridize. The presence of the target sequence in the sample can be determined qualitatively or quantitatively usually by separating hybridized and non-hybridized probe, then determining the amount of labelled probe which hybridized, either by determining the presence of label in probe hybrids, or by determining the quantity of label in non-hybridized probes.

A known labelling technique involves incorporating a radioactive label, such as $^{125}I$ or $^{32}P$ into the probe. Use of radiolabelled probes is associated with certain drawbacks, however, including probe instability and the usual hazards associated with handling radioisotopes. In an effort to overcome these drawbacks, probes have more recently been labeled with non-radioactive tags, such as biotin, haptens, or fluorophores. The utility of such non-radioactive tags has already seen widespread application in the area of immunodiagnostics. Use of non-radioactive tags in detection of DNA probes has also already been reported in the literature. Examples of detection protocols include use of enzyme-antibody conjugates, which recognize a hapten tag, or enzyme-avidin conjugates, which recognize biotin tags.

Early methods developed for attachment of non-radioactive labels to synthetic nucleotide multimer probes involve chemical modifications of the probe after it has been synthesized and/or purified ("post-modification"). For example, R.P. Viscidi et al. (J. Clin. Microbiol., 1986, 23, pp. 311-317) used a bisulfite-catalyzed transamination reaction to attach 1,2-diaminoethane at the C-6 position of cytosine residues in lambda phage DNA. Reaction of the transaminated DNA with biotinyl-e-aminocaproic acid N-hydroxysuccinimide ester, produced biotinylated DNA which could be hybridized to complementary DNA on nitrocellulose and detected using a streptavidin-alkaline phosphatase complex. We have attempted labeling a synthetic DNA oligonucleotide by this method, and have observed a dramatic lowering of the melting temperature associated with its RNA hybrid. Thus, attachment of labels on cytosine appears to interfere with normal base pairing associated with hybridization and may not be a suitable method in the case of short synthetic oligonucleotides.

Another post-modification method involves use of a photo-activatable analog of biotin which randomly labels DNA (A.C. Forster et al., Nucl. Acids Res., 1985, 13, pp. 745-761). Again, this method is not particularly suited to labelling synthetic oligonucleotides due to interference with base pairing and resulting destabilization of the hybrid formed with the target oligonucleotide.

In a further post-modification method, various nucleotide and deoxynucleotide triphosphates have been prepared with amine-terminated linkers which can be incorporated into short DNA probes by enzymatic means. Amine linkers have been substituted at the N6-position of adenine (L. Klevon, PCT application number WO 86/02929, published May 22, 1986, the C5-position of cytosine (D. Ward et al., European Patent publication number 0063879, published March 11, 1982). In one method, a biotinylated form of C5-modified dUTP has been attached to the 3′-end of an oligonucleotide primer with a complementary oligonucleotide as template using the enzyme DNA polymerase (A. Murasugi and R.B. Wallace, DNA, 1984, 3, pp. 269-277; P.R. Langer, A.A. Waldrop and D.C. Ward, Proc. Natl. Acad. Sci. USA, 1981, 78, pp. 6633-6637). Another method employs the use of the enzyme terminal deoxynucleotidyl transferase to add these

linker-modified triphosphates to the 3′-end of oligonucleotides (see, for example, L. Riley et al. DNA, Vol. 5, p. 333, 1986). Drawbacks to these methods include the number of purification steps required, limitations to scale-up inherent in working with pre-purified oligonucleotides, and the potential for mis- or non-pairings associated with the additional or modified nucleotide residues under hybridization conditions.

In another post-modification method, amine linkers have been attached to the 5′-end of the 5′-phosphorylated oligonucleotides using carbonyldiimidazole (B. Chu, G, Wahl and L.E. Orgel, Nucl. Acids Res., 1983, 11, pp. 6513-6529). This method has been used to attach biotin for use in non-radioactive detection formats (B. Chu and L.E. Orgel, DNA, 1985, 4, pp. 327-331; A. Chollet and E.H. Kawashima, Nucl. Acids Res., 1985, 13, pp. 1529-1541). Attachment of a label at the 5′-end has the advantage of generally reducing interference of the probe in hybridization reactions. However, the method still suffers from the number of steps required to attach the linker, namely, enzymatic phosphorylation, reaction with imidazole, reaction with 1,2-diaminoethane or 1,6-diaminohexane, and column purification.

A more direct labelling method is incorporation of the attachment of an amine linker, as a step in automated solid-phase nucleotide multimer synthesis. Of the several methods which have been described in the literature, none is without limitations. Wachter et al. (Nucl. Acids Res., 1986, 14, pp. 7985-7995) used carbonyldiimidazole to achieve aminoalkylation of the 5′-hydroxyl of a synthetic oligonucleotide at the end of a step-wise automated synthesis; the oligonucleotide was still attached to the polymer support. Although this method has the advantage of simplifying purification, the reaction conditions required that the last steps be done manually. Kempe et al. (Nucl. Acids Res., 1985, 13, pp. 45-57) attached 2-(biotinylamido) ethanol to a 5′-phosphorylated synthetic oligonucleotide attached to a solid support in the presence of a condensing agent. In addition to being somewhat laborious, this method requires that the attached label survive the conditions of deprotection and removal from the solid support. Thus, it may not be suited for attachment of chemically sensitive labels.

The procedure of Smith et al. (Nucl. Acids. Res., 1985, 13, pp. 2399-2412) is more directly applicable to automated oligonucleotide synthesis in that a 5′-protected-5′-amino-5′-deoxythymidine-3′-O-phosphoramidite is attached in a final standard coupling reaction. A free amino group is generated during deprotection and removal from the solid support, which is then available for attachment of a variety of labels. Since a nucleotide skeleton (i.e. thymidine) is used, in order to preserve a complimentary relationship between a probe and a target sequence, any of 8 different linkers of the type described by Smith et al. would have to be prepared. Otherwise, an additional thymidine residue could, in some cases, cause a loss in specificity for the target sequence due to mis-hybridization.

Compounds have been suggested which can be used to insert a primary amine-modified digonucleotide during standard automated synthesis procedures. Such compounds include analogs of deoxythymidine, deoxyadenine, and deoxyguanine (G.B. Dreyer et al. Proc. Natl. Acad. Sci. USA, Vol. 82, p. 968, 1985; J.L. Ruth, PCT Application No. WO84/03285 published August 30, 1985). However, these methods suffer from the same limitations as the Smith et al method.

Agrawal et al. (Nucl. Acids Res., 1986, 14, pp. 6227-6245) recently reported two methods of attaching labels which are applicable to automated solid-phase DNA synthesis. In the first method, a uridine-2′,3′-protected-5′-phosphoramidite or 5′- phosphite is added to the 5′-end of the oligonucleotide as a last coupling step. The 2′- and 3′-hydroxyls are subsequently deprotected and oxidized to a dialdehyde using periodate. Biotin hydrazide is then condensed to form a cyclic linkeage after treatment with borohydride. This method suffers from the disadvantage of requiring several post-modification steps. In the second method, an N-protected-O-phosphoramidite derivative of ethanolamine is added in the last coupling cycle during automated synthesis. N-protection is accomplished by means of a 0-fluorenylmethoxycarbonyl groups.

## DEFINITIONS

As used in this disclosure and claims, the following terms are defined as:

nucleotide: a subunit of a nucleic acid consisting of a phosphate group, a 5 carbon sugar and a nitrogen containing base. In RNA the 5 carbon sugar is ribose. In DNA, it is a 2-deoxyribose. The term also includes analogs of such subunits.

nucleotide multimer: a chain of nucleotides linked by phosphodiester bonds or analogs thereof.

oligonucleotide: a nucleotide multimer generally about 10 to about 100 nucleotides in length, but which may be greater than 100 nucleotides in length. They are usually considered to be synthesized from nucleotide monomers, but may also be contained by enzymatic means.

polynucleotide: a nucleotide multimer generally about 100 nucleotides or more in length. These are usually

of biological origin or are obtained by enzymatic means.

nucleotide multimer probe: a nucleotide multimer having a nucleotide sequence complementary with a target nucleotide sequence contained within a second nucleotide multimer, usually a polynucleotide. Usually the probe is selected to be perfectly complementary to the corresponding base in the target sequence. However, in some cases it may be adequate or even desirable that one or more nucleotides in the probe not be complementary to the corresponding base in the target sequence, or that various moieties of synthetic origin either replace a nucleotide within the probe or be inserted between bases of the probe. Typically, the probe is labeled.

oligonucleotide probe: a probe of synthetic or enzymatic origin having less than 100 nucleotides, but which may contain in excess of 200 nucleotides.

hybrization: the formation of a "hybrid", which is the complex formed between two nucleotide multimers by Watson-Crick base pairings between the complementary bases.

## SUMMARY OF THE INVENTION

The present invention then, provides a reagent which is suitable for preparing a 5′ hydroxy substituted nucleotide. The foregoing nucleotide is usually the last nucleotide in a biological or synthetic nucleotide multimer, typically the latter. After the desired sequence has been synthesized employing a standard nucleotide multimer solid phase synthesis, the 5′ hydroxy of the last nucleotide is deprotected, and it may then react with a suitable coupling group on the present reagent to provide a substituted nucleotide multimer. A linking moiety on the reagent is protected by an acyl functional group, which inhibits reagent polymerization during the foregoing coupling step. The linking moiety can be subsequently deprotected under the same non-adverse alkaline conditions typically used to deprotect exocyclic amines, following solid-phase synthesis of a nucleotide multimer. Thus deprotection of the acyl protected linking moiety and exocyclic amines can be accomplished simultaneously, without need of any steps additional to those used in standard nulcleotide solid-phase synthesis. The resulting substituted nucleotide multimer then, has a 5′ "linking arm" which can be linked to useful moieties, such as a non-isotopic labels.

Accordingly, the present invention provides, in its broad aspect, a reagent suitable for preparing a 5′ hydroxy substitued nucleotide (again, typically a terminal nucleotide on a nucleotide multimer), which reagent has the formula:

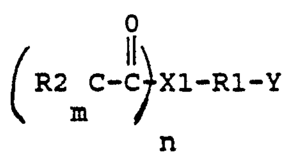

if X1 = sulfur, n = 1

if X1 = amino group, n = 1 or 2

m = either of 1, 2 and 3

In the foregoing formula, Y is a non-nucleotide based coupling group, R1 is a non-nucleotide skeleton, X1 is a protected linking moiety selected from sulfur or an amino group (which includes substituted amino groups in which the amino N is bonded to R1 and the carbonyl carbon of the protecting group). The acyl group

O

$R^2_m$ C-C- , is a protecting group for X1, which can be cleaved (under conditions described below), to leave a linking group H-X1-. By any constituent being "non-nucleotide" is meant that the structure of that constituent is such that it "does not significantly participate in hybridization, said structure must not, for example, participate in any significant hydrogen bonding with a nucleotide, and would exclude structures having as a component one of the 5 nucleotide bases or analogs thereof. Y is selected such that it is capable of coupling R1 to the 5′ hydroxy of a nucleotide, under coupling conditions which are non-adverse. By "non-adverse" conditions, is meant that the conditions are such that they do not substantially adversely affect the polymer backbone and its sugar, base, linker arm or other components, nor the monomeric reagents.

Further, in the above formula, m represents an appropriate number of R2 to satisfy the normal tetravalence of carbon. Each R2 may be part of a cyclic group. Each R2 may also be the same or they may be different, the important consideration being that they are selected such that the alpha carbon of the acyl group, has an electronegativity such that X1 is not substantially deprotected under the coupling conditions

(and thus reagent polymerization is thereby inhibited), but is deprotected under non-adverse alkaline conditions to allow X1 to link to some useful moiety under non-adverse conditions. In this regard, one measure of electronegativity of $R2_m$ is given by the Hammett Equation (for a review on the use of this equation and a table of substituent parameters, see J. Connolly Martin, "Quantitative Drug Design, A Critical Introduction," Marcell Dekker, Inc., NY, 1978):

$$\log (k/ko) = \rho\sigma_p$$

which, as written above, may be defined for the ionization of para-substituted benzoic acids in aqueous solution, in which:

k = equilibrium constant of ionization of para-substituted benzoic acid.
ko = equilibrium constant of ionization of benzoic acid.
$\rho$ = a constant, defined as 1.00 for the ionization of benzoic acids in water at 25° C.
$\sigma_p$ = an empirically determined value for a particular para-substituent.

Positive values of $\sigma_p$ reflect the electron-withdrawing potential of a particular substituent.

In the preferred case of the present invention where each R2 is F (i.e., the protecting group is trifluoroacetyl), $\sigma_p$ (F-) is 0.15, and $\sigma_p$ ($CF_3$-) = 0.54. Thus it would be predicted that other suitable $R2_mC$-groups might include $Cl_3C$-($\sigma_p$ = 0.33), N C ($\sigma_p$ = 0.66), $CF_3CF_2$- ($\sigma_p$ = 0.52), $CH_3C$- ($\sigma_p$ = 0.50), $CH_3O$-C- ($\sigma_p$ = 0.45), or where X1 = N and n = 2, might also include groups which form cyclic N groups.

Several of the amino protecting groups commonly used in the art for protein modifications might also be predicted to serve as suitable amino protecting groups in the present invention. (For a review of protein amino protecting groups, see "The Peptides, Vol. 3, Protection of Functional Groups in Peptide Synthesis", ed. E. Gross and J. Meinhofer, Academic Press, 1981). These may include trifluoracetyl, pthaloyl and maleoyl.

Preferably then, X1 = N, and at least one, and preferably each R2 is selected from a halogen (most preferably each R2 is fluorine). Typically, R1 will be alkyl, desireably being an acyclic hydrocarbon chain of from 1 to 10 carbon atoms.

Preferably, -Y is selected from groups having the formula:

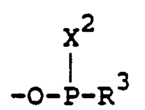

and

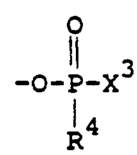

In the foregoing formula:
$X^2$ = halogen (preferably Cl) or substituted amino (preferably a dialkyl amino or heterocyclic N-amine);
$X^3$ = halogen (preferably Cl); amino (preferably a heterocyclic N-aminO); or $O^-$;
$R^3$ = alkyl; preferably an alkoxy selected from methoxy, ethoxy, chlorophenoxy, or betacyanoethoxy); or aryloxy
$R^4$ = alkyl; preferably alkoxy selected from methoxy, ethoxy, or beta-cyanoethoxy); aryloxy, such as chlorophenoxy; or H only if X3 = $-O^-$.

A method of preparing a nucleotide multimer, which has a linking moiety at the 5′ hydroxy position, is also disclosed, which method uses reagents of the type described above and is performed under non-adverse conditions. Broadly, the method comprises first coupling R1 through Y (that is Y serves as the coupling group and all or a portion of Y may or may not remain after actual coupling), to the 5′ hydroxy of a terminal nucleotide in a nucleotide multimer. Then, the resulting product is exposed to non-adverse alkaline conditions, so as to simultaneously deprotect any of the linking moiety and any protected exocyclic amines and phosphates.

## DRAWINGS

Embodiments of the present invention will now be described with reference to the drawings in which:

Fig. 1 illustrates the synthesis of a reagent of the present invention;

Fig. 2 illustrates the synthesis of a substituted nucleotide multimer of the present invention; and

Fig. 3 illustrates a method of labeling the substituted nucleotide multimer produced by the method shown in Fig. 2, by linking a biotin label to the amine linking moiety on the former.

## DETAILED DESCRIPTION OF THE EMBODIMENTS OF THE INVENTION

Examples 1-5 below illustrate various aspects of the present invention. In particular, Example 1 describes in detail the synthesis of a reagent of the present invention. Example 2 describes the synthesis of a substituted nucleotide multimer of the present invention, by a coupling reaction between the reagent produced in Example 1 and a synthetic nucleotide multimer. Example 3 further illustrates the synthesis of other substituted nucleotide multimers of the present invention, while Examples 4 and 5 illustrates linking of a label to such a substituted nucleotide multimer.

### Example 1

Figure 1 illustrates the scheme of the following synthesis.

### Materials:

6-amino-1-hexanol, S-ethyltrifluorothioacetate, N,N-diisopropylethylamine and N,N-diisopropylamino-chloromethoxyphosphine were products of Aldrich Fine Chemicals, Milwaukee, WI.

### (a) 6-Trifluoroacetamido-1-hexanol (IV)

To a stirring slurry of 6-amino-hexanol (II) (1.17g, 10 mmol) in anhydrous ethylacetate (15 ml) was added dropwise S-ethyltrifluorothioacetate (2 ml, 15 mmol). A clear solution was obtained as soon as the addition of S-ethyltrifluorothioacetate was completed. After stirring the reaction mixture at room temperature for 5 hours, 0.5 ml more of S-ethyltrifluorothioacetate was added. After stirring the reaction mixture for another 1/2 hour, silica gel thin-layer chromotography in acetone-dichloromethane, 5:7 ratio, indicated that the starting amino-hexanol (II) had disappeared (Ninhydrin spray). Visualization of the TLC plate after spraying with 0.1 M piperidine followed, after 15 minutes, by ninhydrin spray and heating the plate at 110° C for 2-3 minutes displayed a faster moving broad spot near the solvent front (Rf 0.85). Petroleum ether (60 ml) was added to the reaction mixture and the turbid solution was stored at -20° C for 16 hours to give colorless powder which was washed with cold (-20° C) petroleum ether (3x5 ml). The product was then dried briefly under vacuum and then treated with 5 ml of 10% pyridine-water mixture for 10 minutes to hydrolyze off any O-trifluoroacetyl group which may have been formed resulting in III. The reaction mixture was concentrated to dryness, the residue left was co-evaporated with pyridine (4x10 ml) to give 1.1 g (47%) of compound IV in the form of a gum.

### (b) 6-Trifluoroacetamidohexane-1-N,N-diisopropylaminomethoxyphosphine I

To a stirring solution of compound IV (0.426 g, 2 mMol) in dry THF (10 ml) was added, under argon, N, N-diisopropylethylamine (1.4 ml, 8 mmol) followed by the addition of N, N-diisopropylamino-chloro-methoxyphosphine (0.79 g, 4 mmol). The reaction was stirred at room temperature for 1/2 hour after which the white precipitate was filtered off. The filtrate was diluted with ethylacetate (60 ml) and washed with 5%

sodium carbonate solution (3x15 ml). The organic layer was then dried over $MgSO_4$ and evaporated to give a mobile syrup which was dried under vacuum for 16 hours to give 0.7 g (94%) of desired compound I which was used without any purification. The presence of the desired phosphoramidite (I) was conformed by $^{31}P$ NMR (in $CD_3CN$, trimethylphosphate, external standard): (ppm) 117.4.

Example 2 Automatic Coupling of Amino-Linker I With the Synthetic DNA Fragments

The scheme of Figure 2 outlines the steps involved in the automated synthesis of a nucleotide multimer followed by the automated addition of the amine-linker reagent I. Polymer-support synthesis of a nucleotide multimer with defined sequence was first accomplished using either an Applied Biosystems ("ABI") Model 380A or a Biosearch Model 8600 DNA synthesizer, using a modified version of the manufacturer's synthesis program. This modification makes use of a basic aqueous wash (10% water in 2% pyridine - THF) step prior to the oxidation step (see Figure 2). This modification was included to minimize side reactions in the overall synthesis.

The automated coupling of the amine-linker I was accomplished by loading it as a 0.2 molar solution in acetonitrile into position #6 on the ABI 380A DNA synthesizer. Using the method, the amino-linker was added to the 5′-hydroxyl group of the resin-bound N-protected oligomer in the same way as 5′-dimethoxytrityl-N-protected-nucleoside beta-cyanoethyl-phosphoramidites of general structure V are added. Following a final basic aqueous wash and oxidation step, the resin-bound oligomer was cleaved from the resin by treatment with concentrated aqueous $NH_4OH$ at room temperature for 1 hour. The oligomer was then deprotected by heating the $NH_4OH$ solution at 55° C for 16 hours. This step simultaneously deprotects exocylic amines of the nucleotides, and the linking amine moiety (by cleaving the trifluoroacetyl to leave a primary amine). After evaporating the ammonium hydroxide solution, the crude product was then purified by electrophoresis on a on 12% polyacrylamide -7M urea gel. Bands were visualized by a UV shadowing technique, whereby a fluorescent thin-layer chromatography plate was placed underneath the gel, and a UV lamp was held over the gel. The linker modified oligomer gave a band which migrated slower on the gel than the unmodified oligomer. The linker modified band was then excised from the gel and extracted with 0.1M $NH_4OAc$ (pH7.5) and then desalted through a column of Sephadex G-25 to obtain the pure 5′ hydroxy substituted nucleotide X. Based on relative band intensities from the polyacrylamide gel (UV shadowing method), the coupling of the amine linker was estimated at greater than 90%.

Example 3

Using the above procedure, the following substituted synthetic nucleotide multimers with the amino-linker at the 5′-end, were synthesized and purified. It will be appreciated that substituted nucleotide X, or any of those substituted nucleotides below, could be used as probes to detect a complementary target nucleotide sequence. Of course in such cases, a suitable label would typically be linked to the amine linking moiety.

(a) $NH_2-CH_2-(CH_2)_4-CH_2-O-\overset{O}{\overset{\|}{\underset{O^-}{\underset{|}{P}}}}-O-$ACC AGG TAC AGG AAT ATT AAC C

(b) $NH_2-(CH_2)_5-CH_2-O-\overset{O}{\overset{\|}{\underset{O^-}{\underset{|}{P}}}}-O-$GCT CGT TGC GGG ACT TAA CCC AAC AT

(c) $NH_2-(CH_2)_5-CH_2-O-\overset{O}{\overset{\|}{\underset{O^-}{\underset{|}{P}}}}-O-$ATG TTG GGT TAA GTC CCG CAA CGA GC

(d) $NH_2-(CH_2)_5-CH_2-O-\overset{O}{\overset{\|}{\underset{O^-}{\underset{|}{P}}}}-O-$GAG GAT TCC GCA CAT GTC AAA ACC AGG TAA

In each case, the substituted synthetic oligomer moved slower than the parent oligomer as shown by the UV shadowing of 12% acrylamide-7 M urea gel.

Example 4

To illustrate the suitability of the amine moiety of the foregoing substituted nucleotides, for linking to a label, it was linked to biotin. The scheme for such linking, and subsequent conjugation of the biotin labeled substituted nucleotide, is illustrated in illustrated in Figure 3.

(a) Biotinylation:

To facilitate monitoring, the 5′-amino-linked synthetic deoxyoligonucleotide detailed in Example 3(b) was first labelled at its 3′-OH terminal using alpha-$^{32}$P(TTP) and terminal deoxynucleotidyl transferase by following a standard protocol of Tu and Cohen (Gene 10. 177) The result is compound XI.

For biotinylation, 5 pmoles lyophilized 3′ -$32_p$ labelled probe (XI) was incubated with 0.2M sodium borate (pH 9.0) (5 ml), dimethyl sulfoxide (4 ml) and 30 mmoles biotin-x-NHS (calbiochem-Belving Corp., San Diego. CA, USA) (1 ml) at room temperature for 1 hour. The reaction mixture was then twice ethanol precipitated by adding 250 ml of 0.3M sodium acetate, 750 ml of ethanol and, 20 ug of glycogen. The pellet of the product XIII was dissolved in 20 ml $H_2O$.

(b) Streptavidin - Conjugate:

The verification of the presence of biotin moiety on the foregoing labelled probe XIII was accomplished by conjugation of the biotinylated probe with streptavidin-agarose according to the following procedure:

In two 1.5 ml Eppendorfh tubes was taken 100 ul each of streptavidine-agarose slurry (Bethesda Research Labs, Inc., Gaithersburg, MD, USA) To one tube was added 1 mg of biotin (Calbiochem-Behring Corp., San Diego, CA, USA) in 500 ul of wash buffer (wash buffer consisted of 500 mM phosphate buffer, 2mM EDTA, 0.5 M sodium chloride pH 7.4). After agitating at room temperature for 15 minutes, 2 ul of the solution by labeled probe XII was added and the reaction mixture was agitated at room temperature for 30 minutes.

To the second tube was added 500 ul of wash buffer and 2 ul of the solution of biotinylated probe and stirred at room temperature for 45 minutes.

Both the tubes were then centrifuged and the supernant removed and labelled as S1 and S2. The pelleted agorose gels were washed again with 500 ul of wash buffer and the supernant combined with S1 and S2 respectively. The residues left were suspended in 500 ul each of wash buffer and the amounts of $32_p$ were measured by Cerenkov scintillation counting.

| | CPM | % Bound |
|---|---|---|
| Tube 1 | 1095] | |
| | ] | 6.02 |
| Supernate 1 | 17588] | |
| Tube 2 | 9901] | |
| | ] | 54.4 |
| Supernate 2 | 9231] | |

The results clearly demonstrated the formation of biotin-streptavidin adduct which in turn further illustrated the presence of amino-linkage on the probe.

## EXAMPLE 5

B. Labelling of Amine Linker-Arm Probe with Acridinium Ester and Subsequent Purification

A 25 mM stock solution of acridinium ester (for composition refer to I. Weeks et al., Clin. Chem., Vol. 29, p. 1474, 1983) was prepared in distilled DMSO. The amine linker probe described above in Example 3-(b) was evaporated to dryness in a 1.5 ml conical polypropylene tube. The following cocktail was constructed by adding the following ingredients in the order listed:
3 ul $H_2O$
1ul 1M HEPES (8.0)
4ul DMSO (distilled)
2ul 25mM acridinium ester in DMSO (distilled)
The mixture was vortexed, spun in a microcentrifuge for 2 seconds (to bring the contents to the bottom of the tube), and incubated at 37° C for 20 minutes. At that point, the following components were added to the reaction cocktail in the order listed:
3.0 ul 25 mM acridinium ester in DMSO (distilled)
1.5 ul $H_2O$
0.5 ul 1M HEPES (8.0)
The Cocktail again was vortexed, spun, and incubated an additional 20 minutes at 37° C. The unreacted label was quenched using a 5-fold excess of lysine by adding 5 ul of 0.125M lysine in 0.1M HEPES (8.0), 50% DMSO, and incubated 5 minutes at room temperature.

At this point the acridinium ester-labelled oligomer was purified using the following method. To the 20ul quenched reaction mixture 30ul 3M NaOAc (5.0), 245 ul $H_2O$ and 5ul glycogen was added as a carrier (the glycogen was pre-treated to remove any nuclease activity). The sample was vortexed briefly and 640ul of absolute EtOH was added. The sample was vortexed briefly and incubated on ice 5-10 minutes, then centrifuged 5 minutes at 15,000 rpm in a microcentrifuge. The supernatant was carefully removed and the pellet was redissolved in 20 ul of 0.1M NaOAc (5.0), 0.1% SDS. The sample was further purified by ion-exchanged high performance liquid chromatography (HPLC) as follows: the 20ul redissolved pellet was injected onto a Nucleogen-DEAE 60-7 ion-exchange HPLC column mounted in an IBM (A registered trademark) 9533 HPLC system. All buffers used in the process were made with HPLC grade water, acetonitrile ($CH_3CN$) and sodium acetate (NaOAc), and reagent grade glacial acetic acid (HOAc) and LiCl. Additionally, all buffers were filtered through 0.45um pore size Nylon-66 filters before use. Buffer A was 20mM NaOAc, pH 5.5, 20% $CH_3CN$, and 1 M LiCl. Elution was achieved with a linear gradient from 55% Buffer A, 45% Buffer B to 30% Buffer A, 70% Buffer B in 25 minutes at a flow rate of 1 ml/min. Absorbance at 260 nm was monitored during the run; fractions of 0.5 ml were collected in 1.5 ml conical polypropylene tubes. Immediately after the run, 5ul of 10% SDS was added to each tube followed by vortexing of each tube (this was done to ensure that the acridinium ester-labelled probe did not stick to the walls of the tube). A 0.5ul aliquot was removed from fractions 21-42 and added to 200ul water in a 12x75mm tube (a separate

pipet tip was used for each aliquot to avoid a carryover problem). The chemiluminescence of each aliquot was then determined in a Berthold Clinilumat by automatic injection of 200ul of 0.25N $HNO_3$, 0.1% $H_2O_2$, followed after a 1 second delay by 200ul of IN NaOH and reading of chemiluminescence for 10 seconds.

Fraction 29-33 were EtOH precipitated by adding to each 5ul glycogen, vortexing, adding 1 ml EtOH to each, vortexing, incubating 5-10 minutes on ice, and centrifuging 5 minutes at 15,000 rpm in a microcentrifuge. Each supernatant was carefully removed, each pellet was redissolved in 20ul 0.1M NaOAc, pH5, 0.1% SDS and these separate fractions were then pooled.

Based upon a comparison of UV absorbance and chemiluminescence output, the chemiluminescence specific activity of the labelled probe and the starting label reagent were comparable on a molar basis.

It will be seen then, that reagents have been described which can readily couple to the 5′ hydroxy position of a nucleotide, typically the terminal nucleotide of a nucleotide multimer, to produce a substituted nucleotide multimer with a linking moiety which is readily deprotected under the same conditions as deprotection of exocyclic amines in standard solid-phase synthesis. As illustrated, such reagents can be coupled to a nucleotide multimer as a final step in well known solid-phase synthesis procedures using standard chemistry of such procedures. Furthermore, no special deprotecting step is required of the linking moiety in the resulting substituted nucleotide multimer, since the acyl protecting group, being cleavable under the same alkaline conditions used to deprotect exocyclic nucleotide amines in the multimer, is deprotected simultaneously therewith.

Although specific embodiments of the present invention have been described, variations and modifications to such embodiments will be conceivable to those skilled in the art. Accordingly, the scope of the present invention is not limited by the embodiments described in detail above.

## Claims

1. A reagent suitable for preparing a 5′ hydroxy substituted nucleotide, which reagent has the formula:

$$(R2_m\,C-\overset{O}{\overset{\|}{C}})_n-X1-R1-y$$

wherein:

(a) y is a non-nucleotide based coupling group which is capable of coupling R1 to the 5′ hydroxy of a nucleotide, under coupling conditions which are non-adverse;

(b) R1 is a non-nucleotide skeleton;

(c) X1 is a protected linking moiety selected from S or an amino group;

$R2_mC-\overset{O}{\overset{\|}{C}}-$ is an X1 protecting group, with m being an appropriate number of R2, each of which may be different, and is comprised of one or more atoms of carbon, oxygen, nitrogen or halogen, or a combination thereof, such that $R2_mC$- has an electronegativity so that X1 is not deprotected under the coupling conditions but is deprotected under non-adverse alkaline conditions.

(e) n = 1 when X1 = S; n = 1 or 2 when X1 is an amino group.

2. A reagent as defined in claim 1 wherein X1 = N and at least one of R2 is selected from a halogen.

3. A reagent as defined in claim 1 wherein $R2_mC$ is selected from $Cl_3C$-, N C-, $CF_3CF_2$-, $CH_3\overset{O}{\overset{\|}{C}}$-, and $CH_3O-\overset{O}{\overset{\|}{C}}$-.

4. A reagent as defined in claim 1 wherein $R2_mC-\overset{O}{\overset{\|}{C}}-$ is selected from trifluoroacetyl, phthaloyl and maleoyl.

5. A reagent as defined in claim 1 wherein X′ = N and the reagent formula is:

$$X^2{}_3C-\overset{O}{\overset{\|}{C}}-X1-R1-y$$

in which each X2 is selected from a halogen and $R^1$ = alkyl.

6. A reagent as defined in claim 1 wherein X1 = N and R1 = alkyl, and the reagent formula is selected from:

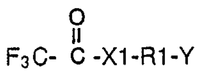

7. A reagent as defined in any one of the preceding claims wherein R1 is an acyclic hydrocarbon chain of from 1 to 10 carbon atoms.

8. A reagent as defined in any one of the preceding claims wherein X is an amino group, and wherein -y is selected from:

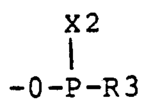 and 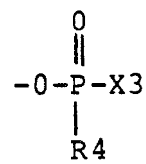

in which:

(a) X2 = halogen or substituted amino
(b) X3 = halogen; amino; or $O^-$
(c) R3 = alkyl; alkoxy; aryloxy;
(d) R4 = alkyl; alkoxy; aryloxy; or H only if X3 = O-.

9. A reagent as defined in any one of claims 1 to 7 wherein R1 = alkyl, X1 is an amino group, and -Y is selected from:

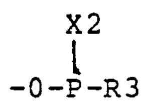 and 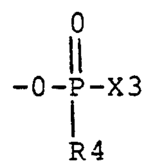

in which:

(a) X2 = Cl or a secondary amino;
(b) X3 = Cl; a secondary amino; or $O^-$
(c) R3 = alkoxy; aryloxy;
(d) R4 = alkoxy; or H only if X3 = O-.

10. A method of preparing a nucleotide multimer with a linking moiety at a 5′ hydroxy, using a reagent of the formula:

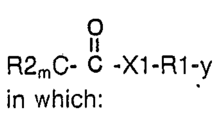

in which:
(i) Y is a non-nucleotide based coupling group;
(ii) R1 is a non-nucleotide skeleton;
(iii) X1 is a protected linking moiety selected from sulfur or an amino;
(iv)
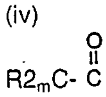 is an X1 protecting group, in which m is an appropriate number of R2 each of which may be different but which is selected such that $R2_mC$-is more electronegative than an alkyl;
the method comprising:

(a) first coupling R1 through Y, to the 5′ hydroxy of a terminal nucleotide of a multimer under non-adverse conditions;

(b) then exposing the resulting product to non-adverse alkaline conditions so as to simultaneously deprotect X1 and any protected exocyclic amines.

11. A method according to claim 10 wherein the reagent is one according to any one of claims 2 to 9 in which n is 1.

12. A method of preparing a labelled nucleotide multimer comprising:

(a) preparing a nucleotide multimer with a linking moiety at a 5′ hydroxy, by the method of claim 10 or 11 and

(b) linking a chemiluminescent acridinium ester to the linking moiety.

180/165
PATENT

**Synthesis of 6-Trifluoroacetamidohexane -1-N, N-diisopropylamino-methoxy-phosphine 1**

FIG. 1

The target compound 1 was synthesized according to the scheme outlined below:

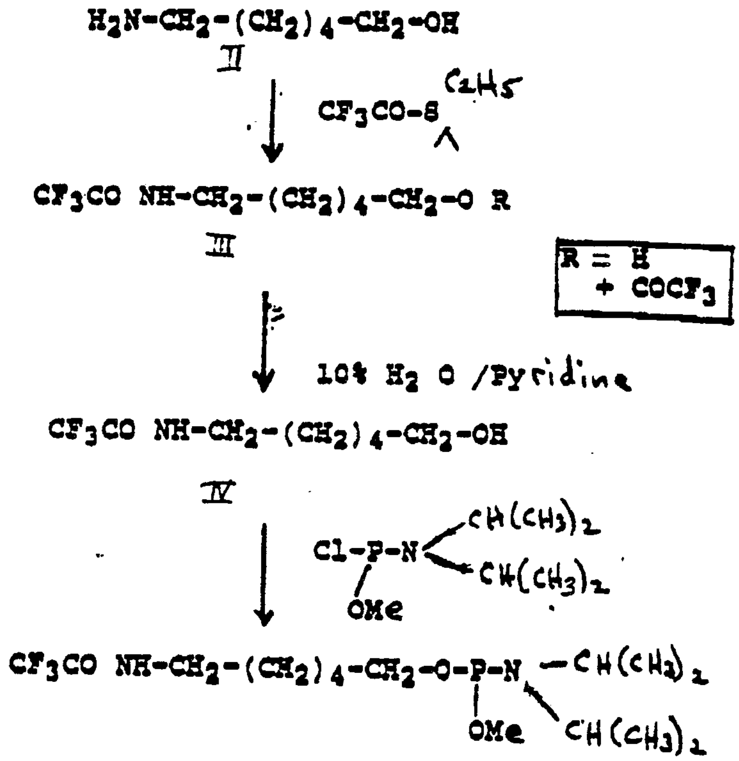


I

Scheme 1

2

Fig 2

180/165
PATENT

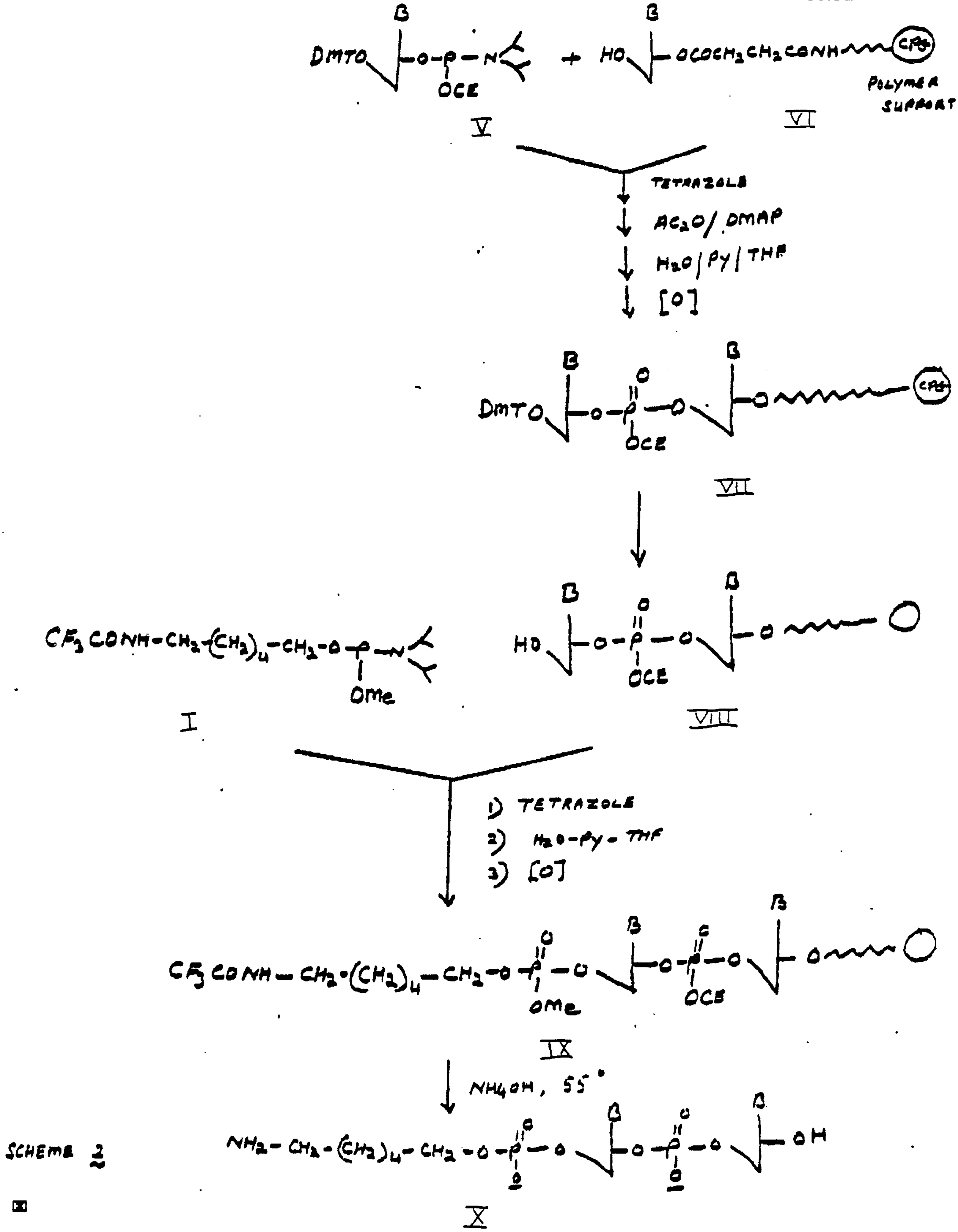


V

VI

VII

VIII

I

IX

X

SCHEME 2

Fig 3.

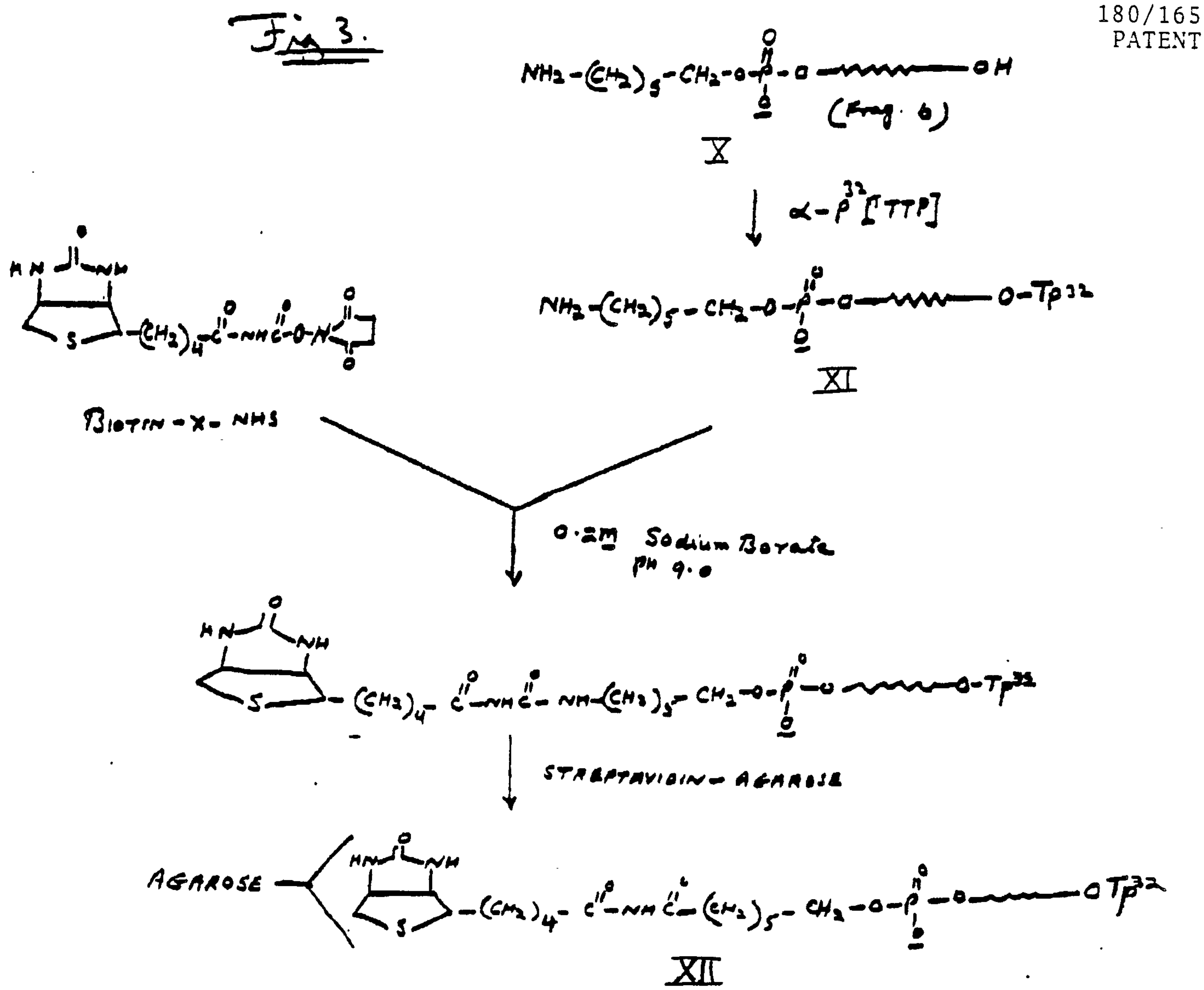
(Frag. 6)
X
α-P32[TTP]
XI
Biotin-X-NHS
0.2M Sodium Borate
pH 9.0
STREPTAVIDIN-AGAROSE
AGAROSE
XII